# EUROPÄISCHE PATENTANMELDUNG

(11) **EP 1 224 948 A1**
(43) Veröffentlichungstag der Anmeldung: **24.07.2002**
(21) Anmeldenummer: 02001355.3
(22) Anmeldetag: 18.01.2002
(51) Int. Cl.: A61L 9/04, A61L 9/14, A61L 9/16, F24F 6/12, B05B 17/06

(54) **Verfahren zur Gasbefeuchtung/Entkeimung**

(30) Priorität: 22.01.2001 DE 20101147 U; 21.12.2001 DE 10163510
(71) Anmelder: Bub AG, 3063 Bern-Itting (CH)
(72) Erfinder: Bolt, Ernst, 3048 Worblaufen (CH); Niederhauser, Thomas, 3176 Neuenegg (CH)
(74) Vertreter: Neidl-Stippler, Cornelia, Dr.

(57) **Zusammenfassung**

Die Erfindung betrifft, Verfahren zur Luftentkeimung, wobei eine Einrichtung zur Gasbefeuchtung/Entkeimung mit einer Gaseinlaßeinrichtung, die Gas in die Einrichtung einbringt; einer Entkeimungsstation mit einem Desinfektionsflüssigkeitsgefäß einer Kavitationseinrichtung am Desinfektionsflüssigkeitsgefäß, die durch Anlegen hochfrequenter Wechselspannung Kavitationen in der im Desinfektionsflüssigkeitsgefäß befindlichen Desinfektionsflüssigkeit erzielt; und einer Aerosolabzugseinrichtung, die das im Raum über dem Desinfektionsflüssigkeitsspiegel des Desinfektionsflüssigkeitsgefäßes gebildete Aerosol in das zu befeuchtende/entkeimende Gas fördert, und einer Gasabgabeeinrichtung, die mit Aerosol versetztes Gas aus der Einrichtung geregelt abgibt, so betrieben wird, daß die Frequenz des Ultraschall-Schwingers in Abhängigkeit der Luftgeschwindigkeit beim Austritt aus dem Vernebelungsgerät so geregelt wird, daß eine in etwa konstante Luftfeuchtigkeit und Aerosoltröpfchengrösseverteilung gebildet wird.

## Beschreibung

Die Erfindung betrifft ein Verfahren zur Gasbefeuchtung/Entkeimung insbesondere für die Luftentkeimung/-befeuchtung. Sie wird nachfolgend hauptsächlich mit Einsatz für Luftentkeimung beschrieben, ist jedoch keineswegs darauf beschränkt, sondern eignet sich mit entsprechender Adaption ebenso für die Entkeimung anderer Gase und Gasmischungen, wie bspw. Narkosegase, Flaschenluft, mit Sauerstoff angereicherte Luft, Stickstoff etc., die für den menschlichen, tierischen oder aber auch pflanzlichen Gebrauch eingesetzt werden soll.

Mikroorganismen, die mit Gasen transportiert bzw. mitgerissen werden können und so zu unerwünschten Infektionen führen, sind mannigfach. Es ist allgemein bekannt, daß in Kliniken, Klimaanlagen etc. häufig Infektionen durch derartige mitgerissene Keime auftreten - Pilzsporen, Pilze, Bakterien, Viren, etc. Das Auftreten derartiger Keime ist in einer Umgebung, in der keine Infektionen auftreten dürfen - so bspw. für Lebensmittelbetriebe, Labors, Kliniken, Produktionsräume für die Kosmetik- und Medikamentenherstellung, Reinraumtechnik, wie für die Chip-Herstellung sowie Schwimmbäder, Gewächshäuser u. dgl., in denen Fremdkeime die Versuche verfälschen würden, unerwünscht. Weiterhin ist die Verteilung von Keimen über Klimaanlagen ein bekanntes Problem und führt unter Umständen zu Masseninfektionen.

Ein weiteres Problem bei der Gasbehandlung besteht darin, daß sehr trockene Gase von Lebewesen als unangenehm betrachtet oder gesundheitsschädlich sind - so wird häufig trockene Luft mittels Befeuchtungsanlagen auf eine verträgliche Luftfeuchte gebracht. Diese Befeuchter sind aber insofern problematisch, als sie über die in ihnen befindliche Flüssigkeit häufig auch darin befindliche Keime verteilen, die in Klimaanlagen entstehen. Es wurde mit verschiedenen Methoden versucht, diese Verkeimung durch die Flüssigkeiten zu verhindern, meist mit toxischen Mitteln, die in vielen Anwendungsfällen unerwünscht sind. Beispielsweise wurde Luft gefiltert, über Laminarauslässe geführt, mit UV bestrahlt oder aber ionisiert. Diese Verfahren sind insofern nicht zufriedenstellend, als sie keine gesteuerte Anfeuchtung der Luft/des Gases der Klimaanlage ermöglichen - was zu einer unangenehmen und für elektrostatische Aufladungen günstigen Atmosphäre führt und außerdem aufwendig ist. Ausserdem können Keime, die auf anderen Wegen als über die Lüftungsanlage in den Raum gelangen, nicht vernichtet werden.

Es sind zwar bereits Luftreinigungsanlagen, die Desinfektionsmittellösungen verdampfen, bekanntgeworden, diese waren jedoch verbesserungsfähig. Bei der Desinfektion über die Luft oder Gase ist es wesentlich, daß das Gas möglichst kleine Desinfektionsmittellösungströpfchen, die weit schweben können, aufweist, die nicht zur Kondensation oder Koaleszenz bereits innerhalb der Anlage neigen. Bereits aus Kostengründen ist es außerdem erwünscht, mit möglichst geringen Desinfektionsmittelmengen eine optimale Wirkung zu erreichen.

Es ist also Aufgabe der Erfindung, ein Verfahren zur Gasbefeuchtung/Entkeimung bereitzustellen, das eine fein dosierbare, auf das Probelm abtgestimmte Desinfektionsmittelmenge in die Atmosphäre einbtringen kann, wobei eine variable kleine Partikelgröße einstellbar sein soll.

Die Aufgabe wird erfindungsgemäß gelöst durch ein Verfahren zur Gasbefeuchtung/Entkeimung, wobei eine Einrichtung zur Gasbefeuchtung/Entkeimung mit: einer Gaseinlaßeinrichtung, die Gas in die Einrichtung einbringt; einer Entkeimungsstation mit einem Desinfektionsflüssigkeitsgefäß, einer Kavitationseinrichtung am Desinfektionsflüssigkeitsgefäß, die durch Anlegen hochfrequenter Wechselspannung Kavitationen in der im Desinfektionsflüssigkeitsgefäß befindlichen Desinfektionsflüssigkeit unter Herstellung eines Aerosols erzielt und einer Aerosolabzugseinrichtung , die das im Raum über dem Desinfektionsflüssigkeitsspiegel des Desinfektionsfiüssigkeitsgefäßes gebildete Aerosol in das zu befeuchtende/entkeimende Gas fördert, und einer Gasabgabeeinrichtung, die mit Aerosol versetztes Gas aus der Einrichtung geregelt abgibt, so betrieben wird, daß die Frequenz des Ultraschall-Schwingers in Abhängigkeit der Luftgeschwindigkeit beim Austritt aus dem Vemebelungsgerät so geregelt wird, daß eine in etwa konstante Luftfeuchtigkeit und Aerosoltröpfchengrösseverteilung gebildet wird.

Vorteilhafte Weiterbildungen ergeben sich aus den abhängigen Ansprüchen.
Die Desinfektionsflüssigkeit oder andere Flüssigkeiten werden in das Desinfektionsflüssigkeitsgefäss gepumpt. Der Flüssigkeitsspiegel im Desinfektionsflüssigkeitsgefäss wird automatisch über Niveausonden geregelt. Durch eine Kavitationseinrichtung ( Piezoschwinger) mit einer regelbaren Wechselspannung - bspw. zwischen 1,1-1,8 MHZ - werden Aerosole in unterschiedlichen Tröpfchengrössen im Desinfektionsflüssigkeitsgefäss gebildet.

Bei niederfrequenter Wechselspannung wird eine geringe Menge von Aerosolen, bei hochfrequente Wechselspannung eine grössere Menge Aerosole produziert. Dadurch wird die gewünschte Vernebelungsmenge reguliert.

Bei geringer Luftgeschwindigkeit im Desinfektionsflüssigkeitsgefäss werden nur die kleineren Aerosoltröpfchen aus dem Desinfektionsflüssigkeitsgefäss abgeführt. Die grösseren Aerosoltröpfchen bleiben im Desinfektionsflüssigkeitsgefäss zurück. Durch die Erhöhung der Luftgeschwindigkeit wird die Vemebelungsmenge, die das Desinfektionsflüssigkeitsgefäss verlassen, erhöht. Somit wird erreicht:
- Max. Wechselspannung und max. Luftgeschwindigkeit =
   100% Vemebelungsleistung mit Aerosolgrössen von 0,3-1,5µm
- Minimale Wechselspannung und minimale Luftgeschwindigkeit =
   3% Vemebelungsleistung mit Aerosolgrössen von ca. 0,3µm

Durch Verändern der Parameter Wechselspannung- Luftgeschwindigkeit, kann die Menge und Tröpfchengrösse der Aerosole beliebig geregelt werden.

Die von der Kavitationseinrichtung (Piezoschwingern) eingebrachte Wärmeleistung ist praktisch unabhängig von obiger Regelung. Bei geringer Leistung und wenig Aerosolen kann die Wärme deshalb nur ungenügend abgeführt werden.

Bisher war äußerst problematisch, daß die Desinfektionsmittellösung in der Kavitationseinrichtung durch das Anlegen einer Wechselspannung an die Ultraschallschwinger, die durch Einbringen von Ultraschallwellen in die Desinfektionsmittellösung das Aerosol herstellen, aufgeheizt wurde. Bei Erwärmung einer Flüssigkeit ändert sich deren Dampfdruck entsprechend und damit auch die Menge und Tröpfchengröße des erzeugten Aerosols. Bei einer zu großen Wärme wird leicht der Taupunkt erreicht und übermäßig gesättigtes Gas produziert, das seine Feuchtigkeit bei Auftreffen auf eine Wand oder im Auslaß durch Kondensation abgibt. Dies ist unerwünscht, da es zu unnötigen Verlusten an Desinfektionsmittellösung und ggf. auch zu Beschädigung von Flächen, an denen Desinfektionsmittel kondensiert, kommen kann. Schließlich ist die Menge so hergestellten Aerosols nicht vorhersehbar und somit keine geregelte gleichmäßige Nebelabgabe möglich. Die Erfindung schlägt nun vor, die Wärme abzuführen, bspw. mittels an der Entkeimungsstation angebrachten Rippen in den Luftstrom abzuführen.

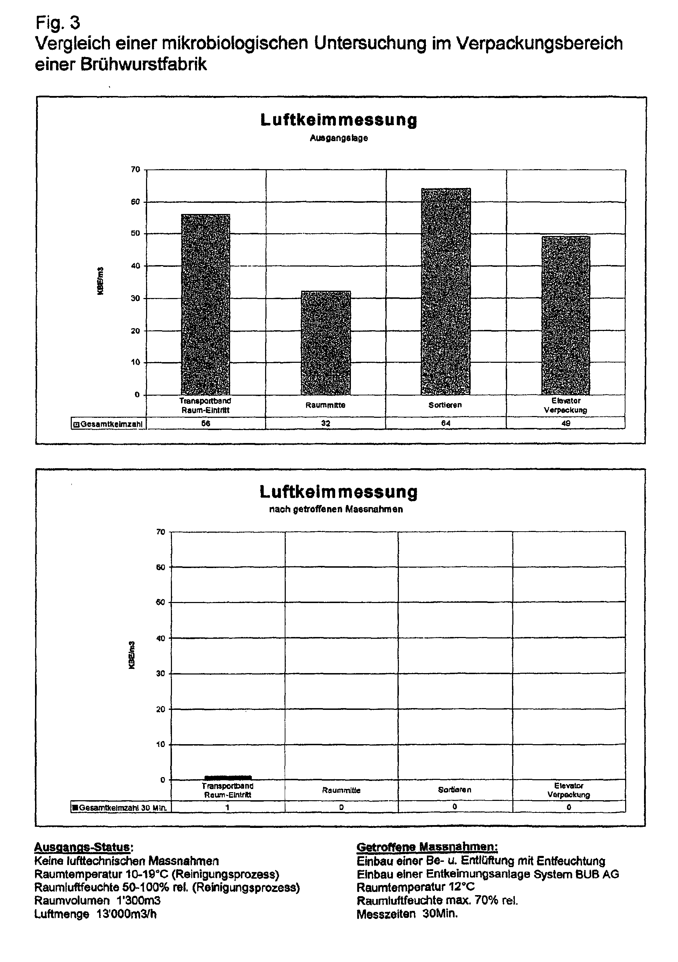

Weiterhin vermeidet das erfindungsgemäße Verfahren eine Erhöhung der relativen Feuchteerhöhung im Kompartement, da bevorzugt nur sehr kleine Mengen Desinfektionsmittel verdampft werden, wodurch u.a. Schimmelbildung und sonstige bekannte, durch zu feuchte Luft auftretende Probleme - vermieden werden können. Somit kann das erfindungsgemäße Verfahren auch in Feuchträumen, wie Schwimmbädern u.dgl., wo eine Trocknung der Luft erwünscht ist eingesetzt werden. Messungen im Verpackungsbereich einer Brühwurstfabrik, in der das erfindungsgemäße Verfahren eingesetzt wurde, haben gezeigt, daß mittels des erfindungsgemäßen Verfahrens die relative Feuchte von Luft nur um weniger als 1% erhöht *werden kann*, während eine sehr gute Entkeimung nach einer Verdüsung von nur 0.02.g Desinfektionsflüssigkeit pro m³/h in Luft erzielt wurde (S. Fig. 3)

Bei einer bevorzugten Ausführungsform als Luftwaschverfahren saugt eine Anlage Umgebungsluft an, versetzt sie mit dem mittels Steuerung des Ultraschallvemeblers und Ventilators unter definierten Bedingungen hergestellten keimwidrigen Aerosol und gibt die so angefeuchtete und entkeimte Luft wieder ab, wodurch das Raumklima unter Vermeidung von Verkeimung befeuchtet und gleichzeitig entkeimt wird.

Es hat sich bei wässrigen Desinfektionsflüssigkeiten als günstig gezeigt, wenn die durch die Kavitationseinrichtung angelegte geregelte Wechselspannung zwischen 1.1 - 1.8 MHz hat besitzt. Bevorzugt ist die Flüssigkeit im Flüssigkeitsaufnahmegefäß eine wäßrige Flüssigkeit - es können aber auch Alkoholelalkoholische Lösungen und andere Flüssigkeiten oder ähnliches eingesetzt werden, solange diese mittels Wechselstrom zu Kavitation angeregt werden können. Das mittels des Verfahrens hergestellte Aerosol besitzt eine Tröpfchengröße im Bereich zwischen 0,3 - 1,5 um, bevorzugt unter 1 µm. Je kleiner die Tröpfchen, um so größer ist die Oberfläche pro Menge Desinfektionsmittel im Tröpfchen und somit steigt die Desinfektionswirkung, die von der wirksamen Desinfektionsmitteloberfläche abhängt, mit fallendem Tröpfchendurchmesser.

Um die Oberflächenspannung des Flüssigkeitsspiegel im Desinfektions-flüssigkeitsgefäss so niedrig wie möglich zu halten, kann bspw. ein Microfaserflies in die Flüssigkeit im Desinfektionsflüssigkeitsgefäss verlegt werden, wodurch die Aerosoltröpfchen dank reduzierter Oberflächenspannung, kleiner werden - es können aber auch andere geeignete Maßnahmen, die dem Fachmann geläufig sind, ergriffen werden.

Durch das erfindungsgemäße Verfahren ist es nun überraschenderweise möglich, die Tröpfchengröße durch die Steuerung der Luftgeschwindigkeit im Desinfektionsflüssigkeitsgefäss Temperatur und des Ultraschallschwingers in einem günstigen Bereich einzustellen und zu halten.

Es ist sinnvoll, Kühleinrichtungen an der Kavitationseinrichtung zur Temperatursteuerung der Desinfektionsmittellösung zu betreiben - durch Kühlung kann das Auftreten größerer Tröpfchen vermieden werden. Geeignete Kühleinrichtungen können bspw. an sich bekannte elektrische (z.B. Peltier Elemente od. dgl.) oder aber strömungstechnische Mittel, wie Kühlrippen od. dgl. am Gefäß sein.

Weiterhin wird erfindungsgemäß das hergestellte Aerosol möglichst schnell in einem Luftstrom verteilt - bspw. durch mehrere Abgabeeinrichtungen - wodurch die Wahrscheinlichkeit des Aufeinandertreffens von Tröpfchen und damit die Bildung größerer Tröpfchen beim Kontakt von zwei kleineren Tröpfchen stark verringert wird und so die günstige geringe Größe der Desinfektionsmitteltröpfchen erhalten werden kann, bis das Aerosol in der Umgebungsluft verteilt und damit die Wahrscheinlichkeit der Koaleszenz von zwei Tröpfchen verringert wird.

Dieser Effekt der Abgabe möglichst kleiner Tröpfchen kann auch dadurch erzielt werden, dass am Austritt des Desinfektions-flüssigkeitsgefässes ein innen mit Rillen versehener Schlauch zu einer Reduzierdüse führt. Sowohl bei beim Schlauch wie bei der Reduzierdüse werden nochmals grössere Aerosoltröpfchen zurückgehalten und ins Desinfektionsflüssigkeitsgefäss zurückgeführt. Das Desinfektionsmittel kann bspw. nach der Reduzierdüse über eine Lanze ( Grösse und Anzahl der Lanzen und deren Schlitzöffnungen oder Rundlöcher, abhängig von der Luftmenge bzw. Desinfektionsflüssigkeitsmenge, in den Luftstrom gebracht werden.

Vorteilhafterweise befindet sich mindestens eine Meßeinrichtung zur Überwachung des Desinfektionsmittelgehaltes in der Abgabeeinrichtung - z.-B. eine Leitwertsonde - Hygrostat - dadurch kann das Verfahren selbstüberprüfend feststellen, ob die erwünschte Herstellung eines Desinfektionslösungsaerosols erfolgt und ggf. entsprechende Maßnahmen bewirken - bspw. Erniedrigung der Geschwindigkeit des Luftstroms, der das gebildete Aerosol aus der Kavitationseinrichtung fördert; Nachdosieren von Desinfektionsflüssigkeit oder aber Auslösen einer Anzeige - wie eines Alarmzeichens auch kann durch eine Überwachung der Flüssigkeitsstandmessung Betriebsniveau 19 mit Vergleich Ist/Sollwert, eine weitere Betriebssicherheit erreicht werden.

Für automatischen Betrieb weist die Vorrichtung einen Flüssigkeitsvorratsbehälter sowie eine mit diesem und dem Desinfektionsflüssigkeitsgefäss verbundene Förderpumpe auf, die Flüssigkeit in das Desinfektionsflüssigkeitsgefäss fördern kann, um dort den Flüssigkeitsnachschub zu sichern. Bevorzugt regelt sich der Nachschub der Desinfektionsflüssigkeit automatisch, indem bei Abfall der Flüssigkeitsmenge in der Kavitationsanlage eine Förderpumpe angesteuert wird, die Flüssigkeit nachliefert - bspw. so lange bis der Füll-Grenzwert erreicht ist.

Es kann auch eine Waage zur Messung des Gewichts des Flüssigkeitsvorratsbehälters verwendet werden, die den Flüssigkeitsvorrat überwacht -selbstverständlich können aber auch andere geeignete Überwachungseinrichtungen für Flüssigkeitspegel, wie sie dem Fachmann geläufig sind, eingesetzt werden, um eine notwendige Nachlieferung von Desinfektionsmittellsöung zu erkennen und ggf. zu veranlassen..

Dadurch, daß durch die erfindungsgemäße Ausgestaltung der Entkeimungsstation eine einfache Entkeimung verschiedenster Bereiche - sowohl solcher mit feuchtigkeitsgesättigter als aus von zu trockener Kompartements möglich ist, kann sicher und schnell ohne großen Aufwand eine Entkeimung durchgeführt werden. Die Anlage eignet sich beispielsweise. für Lebensmittelbetriebe, Labors, Kliniken, Prodluktionsräume für die Kosmetik und Medikamentenherstellung, Reinraumtechnik, wie für die Chip-Herstellung sowie Schwimmbäder, Gewächshäuser und dgl..

Nachfolgend wird die Erfindung anhand der begleitenden Zeichnung einer bevorzugten Ausführungsform der Erfindung näher erläutert, auf die sie aber keineswegs beschränkt ist - diese dient nur der Illustration und dem besseren Verständnis. Darin zeigt:
Fig. 1 ein Fließschema einer Luftentkeimung von Außenluft;
Fig. 2 eine detailliertere schematische Ansicht der Luftentkeimungsanlage, und
Fig. 3 ein Diagramm von Keimbelastungsmessungen in der Lebensmitteltechnologie.

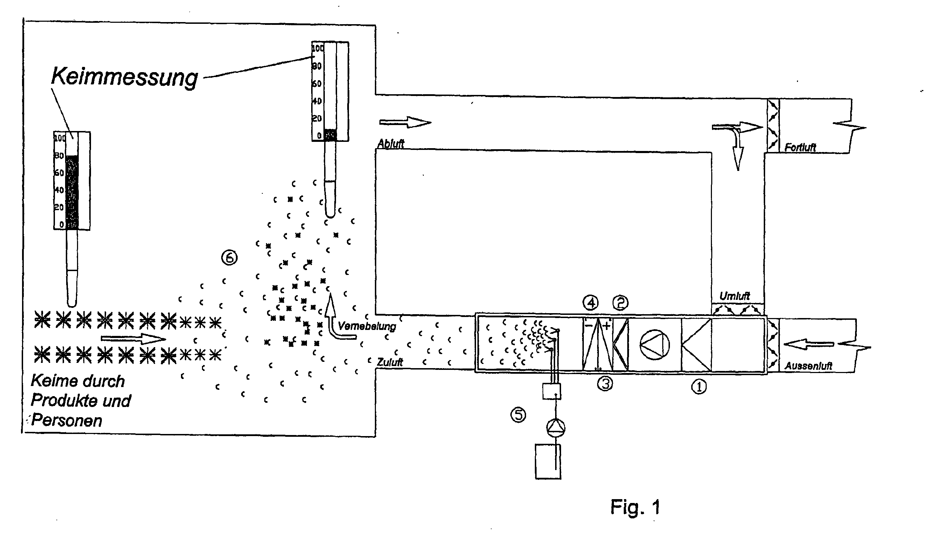

Wie in Fig. 1 gezeigt, wird bei einem Einsatz des erfindungsgemäßen Verfahrens zur Luftreinigung aus einer Entkeimungsstation 5, die hier über mehrere Ausbringlanzen Aerosol in einen vorgefilterten (mittels Grobstaubfilter und Feinstaubfilter 1 und 2) Außenluftstrom abgegeben und in das zu entkeimende Kompartement 6 gebracht. Dort findet eine Keimmessung in an sich bekannter Weise statt, um den Erfolg des Entkeimungsverfahrens zu überprüfen.

Die Außenluft wird über einen Grobstaubfilter 1, einen Feinstaubfilter 2, vorgereinigt, in einem Lufterhitzer 3 und einem Luftkühler 4 konditioniert und mit aus der Entkeimungsstation 5 austretendem Aerosol vereinigt. Diese mit Desinfektionsmittel angereicherte Luft gelangt hier in ein Kompartement 6, das Keime aufweist, die bspw. durch Produkte und Personen auftreten können. Durch eine Keimmessung kann der Keimgehalt der Luft überwacht werden. Die Abluft kann bei dieser Anlage im Kreis geführt werden bzw. ggf. mit desinfektionslösungsangereichterter Außenluft versetzt wieder in den Kreislauf eingebracht werden - es ist aber auch möglich, nur Außenluft zu verwenden und die Abluft vollständig als Fortluft auszuschleusen. Je nach Anwendung kann die im Kreislauf befindliche Luft somit weiterverwendet werden - wie es bspw. dann erwünscht ist, wenn die Luft erwärmt sein soll und die Wärme erhalten werden soll - oder aber es kann ständig neue Außenluft entkeimt und eingesetzt werden. Diese Anlagen eignen sich bspw. zu einer Behandlung der gesamten Atmosphäre eines Kompartements - in Ergänzung zu Reinraumtechniken - oder aber für Teilbereiche - wie mittels Gehäusen abgeschlossene Transportbänder oder aber zur gezielten Einbringung von Reinluft in Füll- und Verschlußbereiche von Abfüllanlagen.

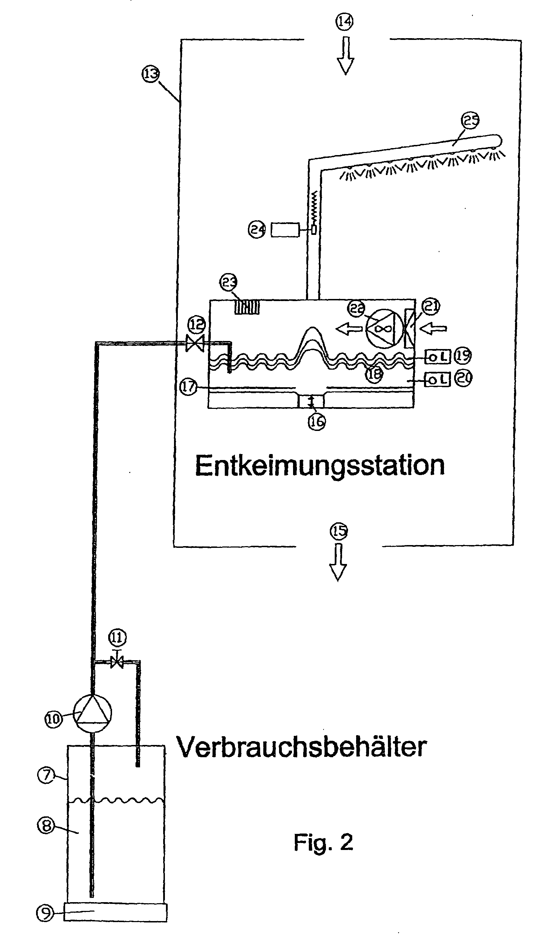

In Fig. 2 ist die Entkeimungsstation 5 der Fig. 1 genauer dargestellt. Hier wird eine Kavitationseinrichtung 16 , die mit Ultraschallschwingern betrieben wird, eingesetzt wobei aus einem Flüssigkeitsvorratsbehälter 6 Desinfektionsflüssigkeit über eine Dosierpumpe 9 entsprechend den Messungen der Flüssigkeitsstandsmeßeinrichtungen 17 und 18 in die Kavitationseinrichtung gefördert und mittels Piezoschwingern (nicht gezeigt) die darin befindliche Desinfektionsflüssigkeit verdampft wird.
Das so erzeugte Aerosol wird mit einem regelbaren Ventilator 22, dem ein Filter 21 vorgeschaltet wird in die Ausbringeinrichtung 25 gefördert, die dieses Aerosol sodann über Lanzen in den Umgebungsluftstrom 14,15 abgibt. Die Ausbringeinrichtung ist hier eine gebogene Lanze mit mehreren Auslaßöffnungen, die eine breitflächige Verteilung des Aerosols ermöglichen. Es können aber auch mehrere Ausbringlanzen oder andere Einrichtungen, die eine möglichst großflächige Abgabe des Aerosols ermöglichen, vorgesehen werden. Bei dieser Lanze ist eine Messsonde 24 in der Aerosoleinbringung 25 vorgesehen, welche die Konzentration des Aerosols mißt.

Ein Flüssigkeitsvorratsbehälter 7 ist mit einem Flüssigkeitsvorrat 8 verbunden, aus dem Flüssigkeit über eine Förderpumpe 10 zur Entkeimungsstation gefördert werden kann, um dort ausreichend Flüssigkeit zur Aerosolbildung vorzuhalten.
Über eine Waage 9 an dem Flüssigkeitsvorrat 8 kann festgestellt werden, ob noch ausreichend Flüssigkeit im Flüssigkeitsvorrat 36 vorhanden ist und ggf. nachdosiert werden. Dies kann über eine hier nicht dargestellte, an sich bekannte Steuerung durchgeführt werden.

Am Desinfektionsflüssigkeitsgefäß 18 ist eine Kavitationseinrichtung 16 - hier in Form eines Piezo-Schwingers .vorgesehen, die Wechselspannung mit einer Frequenz von etwa 1.1-1.8 MHz an die im Desinfektionsflüssigkeitsefäß 18 befindliche Flüssigkeit anlegt. Diese Wechselspannung bewirkt das Auftreten von Kavitationen in der Flüssigkeit, was zur Aerosolbildung im Raum oberhalb des Flüssigkeitsspiegels führt. Das so gebildete Aerosol hat Tröpfchen mit einem Durchmesser von 0.13 - 1,5µm.

Das Desinfektionsflüssigkeitsgefäss 18 wird mittels regelbarem Ventilator 22 durchlüftet. Zum Schutze des Ventilators 22 ist Filter 21 vorgeschaltet. Die Durchlüftungsluftgeschwindigkeit ist variabel zwischen 0,3 - 4m/sec regelbar. Um die Oberflächenspannung des Flüssigkeitsspiegels im Desinfektions-flüssigkeitsgefäss 18 so niedrig wie möglich zu halten, wird ein Microfaserflies 17 in die Flüssigkeit im Desinfektionsflüssigkeitsgefäss 18 verlegt, wodurch die Aerosoltröpfchen dank reduzierter Oberflächenspannung, kleiner werden. Dadurch wird ein Aerosoltröpfchendurchmesser von weniger als 1µm erreicht

Bevorzugt wird hier eine keimhemmende wäßrige Lösung in ein Aerosol überführt, das dann über die Aerosolabzugsein-richtung 25 in die in der Kammer befindliche Luft gebracht wird und diese befeuchtet und gleichzeitig entkeimt. Über die Gasabgabeeinrichtung, die bspw. ein Ventil, eine Pumpe od. dgl. sein kann, wird dann die so gereinigte Luft wieder abgegeben.

Dadurch, daß Gas - bspw. Luft - gleichzeitig entkeimt und gesteuert befeuchtet wird, können durch das erfindungsgemäße Verfahren für sehr viele Einsatzgebiete optimale Klimabedingungen hergestellt werden.

Weitere Ausgestaltungen und Fortentwicklungen sind im Rahmen des Schutzumfangs der Ansprüche dem Fachmann offensichtlich und der Schutzumfang ist keineswegs auf die hier beispielhaft aufgeführten Ausführungsformen begrenzt, die lediglich zur Erläuterung dienen sollen.

### Bezugszeichenliste

- 1.: Grobstaubfilter
- 2.: Feinstaubfilter
- 3.: Lufterhitzer
- 4.: Luftkühler
- 5.: Entkeimungseinheit
- 6.: Kompartement
- 7.: Flüssigkeitsaufnahmegefäss
- 8.: Flüssigkeit
- 9.: Waage
- 10.: Förderpumpe
- 11.: Druckhalteventil
- 12.: Absperrventil
- 13.: Lüftungskanal oder Kanal
- 14.: Gas- bez. Luftstrom Eintritt
- 15.: Gas- bez. Luftsrom Austritt
- 16.: Kavitationseinrichtung
- 17.: Vlies
- 18.: Desinfektionsflüssigkeitsgefäss
- 19.: Flüssigkeitsstandmessung Betriebsniveau
- 20.: Flüssigkeitsstandmessung Trockengeschutz
- 21.: Filter
- 22.: Ventilator
- 23.: Gehäusekühlung
- 24.: Mess Sonde
- 25.: Aerosoleinbringung

## Patentansprüche

1. Verfahren zur Luftentkeimung, wobei eine Einrichtung zur Gasbefeuchtung/Entkeimung mit einer Gaseinlaßeinrichtung, die Gas in die Einrichtung einbringt; einer Entkeimungsstation mit einem Desinfektionsflüssigkeitsgefäß einer Kavita-tionseinrichtung am Desinfektionsflüssigkeitsgefäß, die durch Anlegen hochfrequenter Wechselspannung Kavitationen in der im Desinfektionsflüssigkeitsgefäß befindlichen Desinfektionsflüssigkeit erzielt; und einer Aerosolabzugseinrichtung, die das im Raum über dem Desinfektionsflüssigkeitsspiegel des Desinfektionsflüssigkeitsgefäßes gebildete Aerosol in das zu befeuchtende/entkeimende Gas fördert, und einer Gasabgabeeinrichtung, die mit Aerosol versetztes Gas aus der Einrichtung geregelt abgibt, so betrieben wird, daß
die Frequenz des Ultraschall-Schwingers in Abhängigkeit der Luftgeschwindigkeit beim Austritt aus dem Vernebelungsgerät so geregelt wird, daß eine in etwa konstante Luftfeuchtigkeit und Aerosoltröpfchengrösseverteilung gebildet wird.

2. Verfahren nach Anspruch 1, **dadurch gekennzeichnet, daß** der vom Ultraschallschwinger gebildete Nebel durch mehrere Abgabeeinrichtungen in einen Luftstrom abgegeben wird.

3. Verfahren nach Anspruch 1 oder 2, **dadurch gekennzeichnet, daß** an der Abgabeeinrichtung eine Meßeinrichtung zur Überwachung des Desinfektionsmittelgehaltes der Luft vorgesehen wird.

4. Verfahren nach einem der vorangehenden Ansprüche, **dadurch gekennzeichnet, daß** das Desinfektionsmittel feinverteilt in Tröpfchen von mit einer Größe von 0,3 - 1,5 um, bevorzugt unter 1,0 um - abhängig von der Luftgeschwindigkeit und der an die Kavitationseinrichtung angelegten Frequenz - abgegeben werden.

5. Verfahren nach einem der vorangehenden Ansprüche, **dadurch gekennzeichnet, daß** die an die Kavitationseinrichtung/den Ultraschallschwinger angelegte Wechselspannung zwischen etwa 1,1 - 1,8 MHz entsprechend der gewünschten Aerosolmengen der zu vernebelnden Desinfektionsflüssigkeit geregelt wird.

6. Verfahren nach einem der vorangehenden Ansprüche, **dadurch gekennzeichnet, daß** die Desinfektionsflüssigkeit eine wäßrige Desinfektionsflüssigkeit ist.

7. Verfahren nach einem der vorangehenden Ansprüche, **dadurch gekennzeichnet, daß** aus einem Desinfektionsflüssigkeitsvorrat über eine mit diesem und dem Desinfektionsflüssigkeitsaufnahmegefäß der Entkeimungsstation erbundene Förderpumpe die Desinfektionsflüssigkeit in das Desinfektionsflüssigkeitsgefäß gefördert wird.

8. Verfahren nach einem der vorangehenden Ansprüche, **dadurch gekennzeichnet, daß** bei Abfall der Desinfektionsflüssigkeitsmenge in der Kavitationsanlage die durch die Förderpumpe so lange Desinfektionsflüssigkeit nachgeliefert wird, bis ein Grenzwert erreicht ist.

9. Verfahren nach Anspruch 9, **dadurch gekennzeichnet, daß** über eine Waage, zur Messung des Gewichts des Desinfektionsflüssigkeitsgefäßes die Desinfektionsflüssigkeitsmenge darin überwacht wird.

10. Verfahren nach einem der vorangehenden Ansprüche, **dadurch gekennzeichnet, daß** ein geregelter Luftstrom von 0,5 - 3.0 m/sec. erzeugt wird, dessen Geschwindigkeit entsprechend der gewünschten Aerosoltröprchengrösseverteilung geregelt wird.

11. Verfahren nach einem der vorangehenden Ansprüche, **dadurch gekennzeichnet, daß** die Oberflächenspannung der zu vernebelnden Desinfektionsmittellösung verringert wird

12. Verfahren nach einem der vorangehenden Ansprüche, **dadurch gekennzeichnet, daß** größere Aerosoltröpfchen mechanisch zurückgehalten werden.

13. Verfahren nach einem der vorangehenden Ansprüche, **dadurch gekennzeichnet, daß** Kühleinrichtungen an der Kavitationseinrichtung zur Temperatursteuerung der Desinfektionsmittellösung vorgesehen werden.

14. Verfahren nach einem der vorangehenden Ansprüche, **dadurch gekennzeichnet, daß** an der Austrittseinrichtung Messeinrichtungen den Entkeimungsmittelgehalt des austretenden Aerosols überwachen.
